# EUROPÄISCHE PATENTSCHRIFT

(11) **EP 0 537 490 B1**
(45) Veröffentlichungstag und Bekanntmachung des Hinweises auf die Patenterteilung: **12.02.1997**
(21) Anmeldenummer: 92115881.2
(22) Anmeldetag: 17.09.1992
(51) Int. Cl.: C12Q 1/56

(54) **Funktioneller Test und Reagenz zur Bestimmung von Fibrinogen**
Functional test and reagent for determining fibrinogen
Test fonctionnel et réactif pour déterminer du fibrinogène

(30) Priorität: 14.10.1991 DE 4133946
(43) Veröffentlichungstag der Anmeldung: 21.04.1993
(73) Patentinhaber: BEHRINGWERKE Aktiengesellschaft, 35001 Marburg (DE)
(72) Erfinder: Fickenscher, Karl, W-3550 Marburg 8 (DE)

(56) Entgegenhaltungen:
- EP-A- 0 137 269
- EP-A- 0 336 353
- EP-A- 0 456 152
- CLINICAL CHEMISTRY. Bd. 29, Nr. 4, April 1983, WASHINGTON, US Seiten 614 - 617 SIEFRING ET AL. 'Development and analytical performance of a functional assay for fibrinogen on the Du Pont aca analyzer'
- PROCEEDING OF THE NATIONAL ACADEMY OF SCIENCES Bd. 75, Nr. 7, Juli 1978, WASHINGTON DC Seiten 3085 - 3089 LAUDANO ET AL. 'Synthetic peptide derivatives that bind to fibrinogen and prevent the polymerization of fibrin monomers'

## Beschreibung

Die Erfindung betrifft ein Verfahren sowie ein Reagenz zur Bestimmung von Fibrinogen aus unverdünnten Plasmaproben.

Fibrinogen ist ein Glykoprotein mit einem Molekulargewicht von 340000. Proteolytische Abspaltung von Fibrinopeptid A und B durch Thrombin führt zur Bildung von Fibrinmonomeren, die zu Fibrin aggregieren. Dieser letzte Schritt in der Gerinnung des Blutes ist essentiell, da er die Bildung des Gerinnsels darstellt. Die Konzentration an Fibrinogen ist sehr variabel. Sie kann durch Verbrauch absinken (erworbener Fibrinogenmangel), sie kann aber auch in der akuten Phase einer Erkrankung, z.B. nach Verbrennungen, stark erhöht sein. Aufgrund seiner bedeutenden Funktion für die plasmatische Gerinnung ist Fibrinogen das am häufigsten bestimmte Protein in der Gerinnungsdiagnostik. Neuere Untersuchungen zeigen, daß chronisch erhöhte Konzentrationen an Fibrinogen mit einer erhöhten Wahrscheinlichkeit von cardiovasculären Erkrankungen korrelieren (Cook, N.S. & Ubben D. 1990, TIPS 11: 444-451; Cooper J. & Douglas A.S. 1991, Fibrinolysis 5: 105-108).

Es sind eine Reihe von Verfahren bekannt, Fibrinogen zu bestimmen.

Verschiedene Methoden der immunologischen Bestimmungen sind bekannt, lassen aber keinen Rückschluß auf die Funktionsfähigkeit der gefundenen Moleküle zu.

Ebenfalls bekannt sind Verfahren, die Fällungsreaktionen mit verschiedenen Reagenzien benutzen (Macart, M. et al. 1989, Clin. Chem. 35: 211-214). Da hier kein Gerinnsel durch Thrombin erzeugt wird, kann ebenfalls nicht die funktionelle Aktivität bestimmt werden. Außerdem sind die Fällungsreaktionen sehr unspezifisch, so daß andere Proteine miterfaßt werden und so das Ergebnis verfälschen können. Diese Verfahren haben keinen Eingang in die Routinediagnostik gefunden und sind ebenfalls nicht Gegenstand der Erfindung.

Die Methoden zur Bestimmung von funktionsfähigem Fibrinogen können in zwei wesentliche Gruppen unterteilt werden:
1. Verfahren, bei denen die Gerinnungszeit eines verdünnten Plasmas bestimmt wird. Diese ist vom Fibrinogengehalt der Probe abhängig. Die zu bestimmende Probe muß daher soweit vorverdünnt werden, daß meßbare Gerinnungszeiten erzielt werden (Clauss, A. 1957, Acta Haemat., 17: 237-246; Vermylen C. et al. 1963, Clin. Chim. Acta 8: 418-424). Bei dieser Methode korrelieren steigende Mengen an Fibrinogen mit abnehmender Gerinnungszeit.
2. Verfahren, bei denen die Menge an erzeugtem Gerinnsel gemessen wird. Das kann z. B. dadurch geschehen, daß das Gerinnsel aus dem Testansatz isoliert, gewaschen und die darin enthaltene Menge Protein bestimmt wird (Ratnoff, O.D. und Menzie, G.A.B., 1951, J. Lab. Clin. Med. 37:316 -3 20). Dieses Verfahren ist sehr arbeits- und zeitaufwendig und wird daher nicht in der Routinediagnostik durchgeführt.
   Auf optischen Systemen wird häufig der insgesamt erreichte Anstieg der optischen Dichte oder der Lichtstreuung beim Eintritt der Gerinnung gemessen (Inada Y. et al. 1978, Clin. Chem. 24: 351-353; Denegri E. & Prencipe L. 1982, Clin. Chem. 28: 1502-1505).

Verwandt mit letzterer Methode ist ein Verfahren welches zur Umsetzung vom Fibrinogen ein thrombinähnliches Schlangengiftenzym von Mitgliedern der Gattung Agkistrodon einsetzt (EP 0 137 269). Zur Auswertung ist ein photometrisches System erforderlich, da die Aggregationsrate bestimmt werden muß. Die Rate des Trübungsanstieges ist ein Maß der Fibrinogenkonzentration, ähnlich wie bei den oben aufgeführten Methoden bei denen der gesamte Signalanstieg zur Auswertung herangezogen wird. Außerdem kann hier der Zeitpunkt des Trühungsanstieges als Maß für Fibrinogenspaltprodukte herangezogen werden.

Besonders nachteilig an dem unter 1. aufgeführten Verfahren gemäß dem Stand der Technik (Clauss, A. (1957)) ist der Umstand, daß die Proben vorher in einem zusätzlichen Arbeitsschritt etwa 1 : 10 verdünnt werden müssen. Werden unverdünnte Proben eingesetzt, so werden durch die notwendigerweise hohen Konzentrationen an Thrombin extrem kurze und nicht mehr auswertbare Gerinnungszeiten erhalten. Bei sehr hohen Fibrinogenkonzentrationen ist oft sogar noch ein zweiter Verdünnungsschritt und eine Wiederholungsmessung notwendig.

Würde in dieser Methode auf die Vorverdünnung verzichtet und zur Vermeidung unbrauchbar kurzer Gerinnungszeiten die Thrombinmenge herabgesetzt, mißt man Verlängerungen der Gerinnungszeit sowohl bei geringer wie bei erhöhter Fibrinogenkonzentration, so daß die gemessene Gerinnungszeit keiner Konzentration mehr zugeordnet werden kann.

Ein weiterer Nachteil der Methode nach dem Stand der Technik ist, daß aufgrund der hohen Verdünnung nur ein sehr schwaches und kleines Gerinnsel entstehen kann. Mechanisch messende Geräte können dieses zwar erfassen, zeigen aber im Vergleich zu den Gerinnungstests, in denen unverdünntes Plasma eingesetzt wird, eine schlechte Präzision. Die heute zunehmend eingesetzten, photometrisch arbeitenden Geräte können sehr häufig die schwachen Gerinnsel nicht mehr zuverlässig erfassen. Daher wird auf diesen Geräten häufig nach anderen, an sich weniger günstigen und weniger aussagekräftigen Verfahren gearbeitet.

Methoden die ein Gerinnsel aufarbeiten, um die enthaltene Fibrinogenmenge zu erfassen, sind zu arbeitsaufwendig und daher nicht routinetauglich.

Verfahren, die turbidimetrisch oder nephelometrisch arbeiten, sind von der Eigentrübung der Proben abhängig und liefern daher nicht immer zuverlässige Ergebnisse; sie sind auf speziell dafür ausgelegte Geräte beschränkt, was ihre allgemeine Anwendbarkeit einschränkt.

Der Erfindung lag die Aufgabe zugrunde, ein Verfahren zu finden, das die Möglichkeit bietet, mit den im Gerinnungs-Routinelabor gängigen Instrumenten Fibrinogen zu bestimmen, ohne daß die Probe vorbehandelt werden muß.

Überraschenderweise wurde gefunden, daß dies erreicht werden kann, indem man die Aggregation des Fibrins durch einen spezifischen Inhibitor partiell hemmt. Die Gerinnung des Fibrins ist an sich gleichbedeutend mit der Aggregation der Fibrinmonomere, jedoch kann durch die geeignete Wahl der Konzentration des Inhibitors die Gerinnungszeit so eingestellt werden, daß Proben sowohl von sehr hoher als auch von sehr niedriger Konzentration in einem praktikabel meßbaren Zeitrahmen koagulieren. Darüberhinaus wurde erreicht, daß die Differenz der Gerinnungszeit zwischen zwei gegebenen Fibrinogenkonzentrationen deutlich größer als im Stand der Technik geworden ist. Die damit besser gespreizte Bezugskurve trägt wesentlich zu einer präzisen Bestimmung des Fibrinogens bei.

In dem erfindungsgemäßen Verfahren kann ein hoher Überschuß an Thrombin oder einer Protease mit analoger Aktivität wie Batroxobin (eine Protease aus dem Gift der Schlange Agkistrodon rhodostoma) eingesetzt werden, so daß alles Fibrinogen sofort in lösliches Fibrin umgewandelt wird. Dadurch hängt die Gerinnungszeit nur noch von der Aggregationsgeschwindigkeit des Fibrins ab. Diese ist bei konstanter Konzentration des Inhibitors eine Funktion der Fibrinkonzentration.

Geeignete Inhibitoren sind Peptide, die in der Struktur analog zum aminoterminalen Ende der human Fibrin α-Kette sind. Diese Peptide, wie auch die dadurch erreichbare Inhibition der Fibrinaggregation, sind an sich bekannt (Laudano A. P. et. al. Proc. Natl. Acad. Sci. USA (1978), 3085-3089; DE 40 14 655).

Solche Inhibitoren haben auch Eingang in verschiedene Gerinnungs-Testsysteme gefunden, in denen die völlige Hemmung der Gerinnselbildung notwendig ist (Miragla C.C. et al. Anal. Biochem. (1985), 144, 165-171, DE 38 11 647). Sie werden immer in einem hohen Überschuß eingesetzt, um Störungen des eigentlichen, vom Clotinhibitor selbst unabhängigen Test, zu vermeiden.

In dem erfindungsgemäßen Verfahren wird die Konzentration so eingestellt, daß die Gerinnungszeit eine praktikable Messung der Fibrinkonzentration zuläßt, bevorzugterweise sollte die Gerinnungszeit bei einer Fibrinkonzentration von 1 g/l etwa 50 bis 150 s betragen.

Bevorzugterweise wird in dem erfindungsgemäßen Verfahren in der Gegenwart eines wasserlöslichen Polyalkohols, z.B. Polyethylenglykol 6000, gearbeitet. Dieser bewirkt, daß auch geringe Fibrinkonzentrationen noch aggregieren und somit meßbar sind. Bevorzugt ist es ferner das Verfahren in Gegenwart eines Inhibitors für Heparin, wie beispielsweise Polybren (Hexadimethrin-Bromid), Protaminsulfat oder -chlorid durchzuführen. Damit kann verhindert werden, daß das in heparinisierten Proben enthaltene AT III in Verbindung mit dem Heparin das im Reagenz enthaltene Thrombin hemmt und so den Test negativ beeinflußt.

Als Inhibitor der Fibrinaggregation werden bevorzugterweise die in der DE-A-40 14 655 beschriebenen Peptide verwendet.

Gegenstand der Erfindung ist somit ein Verfahren zur Bestimmung von Fibrinogen, wobei die Probe unverdünnt eingesetzt wird und ein Inhibitor der Fibrinaggregation eingesetzt wird.

Ferner ist Gegenstand der Erfindung ein Verfahren wie oben beschrieben, wobei die Gerinnungszeit gemessen wird.

Weiterhin ist Gegenstand der Erfindung ein Verfahren wie oben beschrieben, wobei die Gerinnung durch Zusatz von Thrombin oder einer analog wirksamen Protease ausgelöst wird.

Ferner ist Gegenstand der Erfindung ein Verfahren wie oben beschrieben, wobei im Falle der Verwendung von Thrombin dieses in einem Überschuß von mindestens 20 U pro ml Plasma zugegeben wird.

Ein weiterer Gegenstand der Erfindung ist ein Verfahren wie oben beschrieben, wobei Probe und Reagenz in einem Verhältnis von 1:1 bis 1:5 gemischt werden.

Gegenstand der Erfindung ist weiterhin ein Reagenz, das bevorzugterweise 10-200 U/ml Thrombin, 20-1000 µg/ml des Aggregationsinhibitors, 0,02-0,8 % eines wasserlöslichen Polyalkohols, 50 bis 250 mM Kochsalz, 20 - 100 mM eines Puffers von pH 7,0 bis 8,5, 2 bis 25 mM Calciumchlorid, 2 bis 100 µg/ml einer Heparin neutralisierenden Substanz sowie Füllstoffe enthält.

Füllstoffe sind z. B. Zucker, Zuckeralkohole, Aminosäuren, hydrisiertes Collagen oder Albumin (wie z. B. Saccharose, Mannit, Glycin, Polygeline).

Besonders bevorzugt wird ein Reagenz, das 30-200 U/ml Thrombin, 100-500 µg/ml eines Aggregationsinhibitors mit der Aminosäuren-Sequenz G-P-R-P-A-amid, 0,06-0,1 % Polyethylenglykol 6000, 100 bis 150 mM NaCl, 50 mM Tris pH 7, 8 - 8,3, 10 mM CaCl₂, 10-20 µg/ml Polybren sowie 1% Rinderserumalbumin enthält.

Eine typische Durchführung des erfindungsgemäßen Verfahrens kann wie folgt aussehen:

Zu einer Probe, z. B. Citratplasma, das auf vorzugsweise 37 °C temperiert wird, wird das 1 bis 5fache, vorzugsweise das 2fache Volumen des erfindungsgemäßen Reagenz zugegeben.

Die Gerinnungszeit wird mit an sich bekannten Meßverfahren bestimmt.

Die folgenden Beispiele sollen die Erfindung näher erläutern:

### Beispiel 1:

### Herstellung eines geeigneten Reagenzes:

Die folgenden Substanzen werden in der angegebenen Konzentration in Wasser gelöst und der pH-Wert eingestellt. Die Lösung ist dann gebrauchsfertig.

200 µg/ml Aggregationsinhibitor (G-P-R-P-A-amid), 50 U/ml Thrombin vom Rind, 0,08 % Polyethylenglykol 6000, 110 mM NaCl, 15 µg/ml Polybrene, 1 % Rinderserumalbumin, 10 mM CaCl₂, 50 mM Tris, pH 8,0.

### Beispiel 2:

Durchführung einer Fibrinogenbestimmung an verschiedenen Geräten

100 µl Citrat-Plasma wurden vorgelegt und auf 37°C temperiert, 200 µl Reagenz nach Beispiel 1 (37°C) wurden zugegeben.

Die Tabelle 1 zeigt die Gerinnungszeiten die an Geräten mit verschiedenen Detektionsverfahren für den Gerinnungszeitpunkt bestimmt wurden.

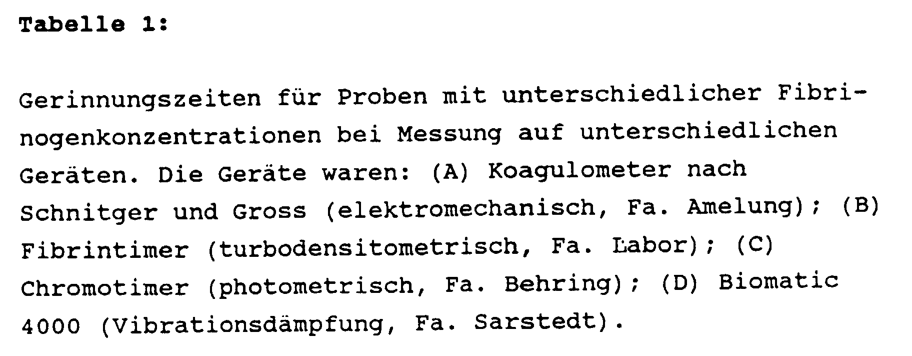

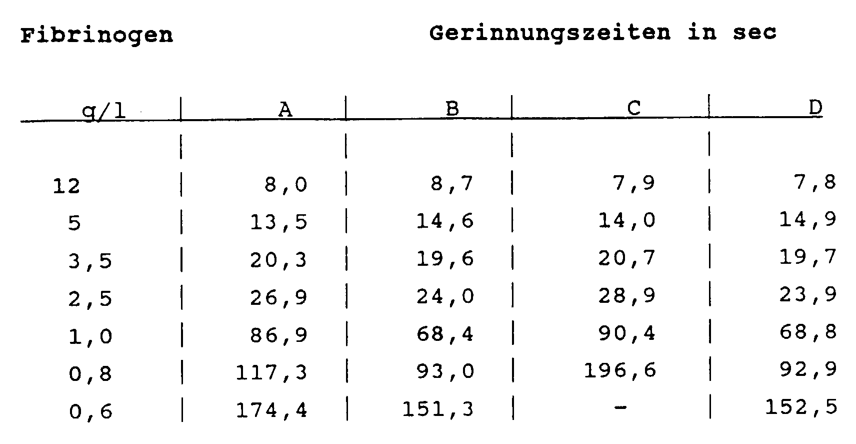

### Beispiel 3

Vergleich der Bezugskurve nach dem erfindungsgemäßen Verfahren und dem Verfahren nach dem Stand der Technik mit Probenvorverdünnung

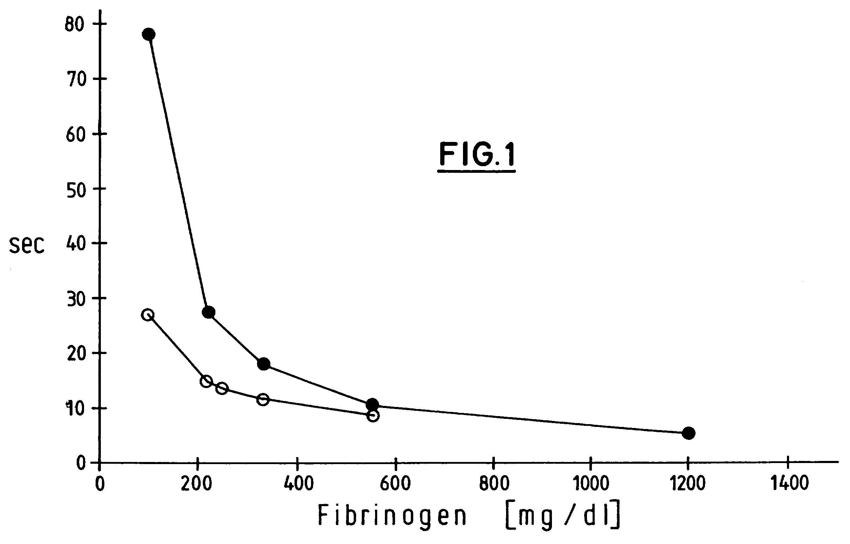

Proben unterschiedlicher Fibrinogenkonzentrationen wurden an einem Gerät (Fibrintimer 2-Kanal, Behring-Werke) mit dem erfindungsgemäßen Verfahren sowie mit einem kommerziell erhältlichen Test (Multifibren^{R}, Behringwerke) gemessen. Bei dem kommerziell erhältlichen Verfahren wird die Plasmaprobe 1:10 mit Puffer vorverdünnt. Zu 200 µl der verdünnten Probe werden nach 1 min Inkubationszeit 100 µl des Reagenzes gegeben und die Gerinnungszeit bestimmt. Die Fig. 1 zeigt die mit beiden Verfahren erhaltene Gerinnungszeiten in Abhängigkeit von der Fibrinogenkonzentration der Probe. (o-o) Verfahren nach Stand der Technik; (●-●) erfindungsgemäßes Verfahren. Bei dem bisher üblichen Verfahren lassen sich Proben im besonders hohen Bereich nicht mehr erfassen, die Datenpunkte fehlen daher.

## Patentansprüche

1. Verfahren zur Bestimmung von Fibrinogen nach bekannten Verfahren, dadurch gekennzeichnet, daß die Probe unverdünnt und ein Inhibitor der Aggregation der Fibrinmonomeren eingesetzt wird.

2. Verfahren nach Anspruch 1, dadurch gekennzeichnet, daß die Gerinnungszeit gemessen wird.

3. Verfahren nach Anspruch 1, dadurch gekennzeichnet, daß die Gerinnung durch Zusatz von Thrombin oder einer analog wirksamen Protease ausgelöst wird.

4. Verfahren nach Anspruch 3, dadurch gekennzeichnet, daß im Falle der Verwendung von Thrombin dieses in einem Überschuß von mindestens 20 U pro ml Plasma zugegeben wird.

5. Verfahren nach Anspruch 1, dadurch gekennzeichnet, daß Probe und Reagenz in einem Verhältnis von 1:1 bis 1:5 gemischt werden.

6. Verfahren nach Anspruch 1, dadurch gekennzeichnet, daß nur ein Reagenz eingesetzt wird.

7. Verfahren nach Anspruch 1, dadurch gekennzeichnet, daß es ein Peptid als Inhibitor der Aggregation der Fibrinmonomeren enthält.

8. Verfahren nach Anspruch 3, dadurch gekennzeichnet, daß die Protease humanen, tierischen oder rekombinanten Ursprungs ist.

9. Reagenz zur Bestimmung von Fibrinogen, dadurch gekennzeichnet, daß 10-200 U/ml Thrombin, 0,02-0,8 % eines wasserlöslichen Polyalkohols, 50 bis 250 mM Kochsalz, 20 - 100 mM eines Puffers von pH 7,0 bis 8,5, 2 bis 25 mM Calciumchlorid, 2 bis 100 µg/ml eines Heparin-Neutralisators, 20-1000 µg/ml ein die Aggregation von Fibrinmonomeren inhibierendes Peptid sowie Füllstoffe enthalten sind.

## Claims

1. A method for determining fibrinogen by known methods, wherein the sample is employed undiluted, and an inhibitor of fibrin monomer aggregation is employed.

2. The method as claimed in claim 1, wherein the coagulation time is measured.

3. The method as claimed in claim 1, wherein the coagulation is induced by addition of thrombin or of a protease with analogous activity.

4. The method as claimed in claim 3, wherein when thrombin is used it is added in an excess of at least 20 U per ml of plasma.

5. The method as claimed in claim 1, wherein sample and reagent are mixed in a ratio of from 1:1 to 1:5.

6. The method as claimed in claim 1, wherein only one reagent is employed.

7. The method as claimed in claim 1, which contains a peptide as inhibitor of fibrin monomer aggregation.

8. The method as claimed in claim 3, wherein the protease is of human, animal or recombinant origin.

9. A reagent for determining fibrinogen, which contains 10-200 U/ml thrombin, 0.02-0.8% of a water-soluble polyalcohol, 50 to 250 mM sodium chloride, 20-100 mM of a buffer of pH 7.0 to 8.5, 2 to 25 mM calcium chloride, 2 to 100 µg/ml of a heparin neutralizer, 20-1000 µg/ml of a peptide which inhibits fibrin monomer aggregation, and bulking agents.

## Revendications

1. Procédé pour la détermination du fibrinogène selon des méthodes connues, caractérisé en ce que l'échantillon est non dilué et on ajoute un inhibiteur de l'agrégation des monomères de fibrine.

2. Procédé selon la revendication 1, caractérisé en ce que l'on mesure le temps de coagulation.

3. Procédé selon la revendication 1, caractérisé en ce que l'on déclenche la coagulation par addition de thrombine ou d'une protéase à activité analogue.

4. Procédé selon la revendication 3, caractérisé en ce que, dans le cas de l'utilisation de thrombine, on l'ajoute en un excès d'au moins 20 U/ml de plasma.

5. Procédé selon la revendication 1, caractérisé en ce que l'échantillon et le réactif sont mélangés en un rapport de 1:1 à 1:5.

6. Procédé selon la revendication 1, caractérisé en ce que l'on n'utilise qu'un seul réactif.

7. Procédé selon la revendication 1, caractérisé en ce que l'on utilise un peptide en tant qu'inhibiteur de l'agrégation des monomères de fibrine.

8. Procédé selon la revendication 3, caractérisé en ce que la protéase est d'origine humaine, animale ou recombinante.

9. Réactif pour la détermination du fibrinogène, caractérisé en ce que son contenu est 10-200 U/ml de thrombine, 0,02-0,8 % d'un polyalcool soluble dans l'eau, 50 à 250 mM de chlorure de sodium, 20-100 mM d'un tampon à pH 7,0-8,5, 2 à 25 mM de chlorure de calcium, 2 à 100 µg/ml d'un neutralisateur d'héparine, 20-1000µg/ml d'un peptide inhibant l'agrégation de monomères de fibrine, ainsi que des excipients.
